## Europäisches Patentamt

## European Patent Office

## Office européen des brevets

(19)

(11) Veröffentlichungsnummer: **0 179 343**
**B1**

(12) **EUROPÄISCHE PATENTSCHRIFT**

(45) Veröffentlichungstag der Patentschrift: **19.09.90**

(51) Int. Cl.⁵: **C 08 G 18/78, B 01 J 13/00**

(21) Anmeldenummer: **85112658.1**

(22) Anmeldetag: **07.10.85**

(54) **Verfahren zur Herstellung von Harnstoffgruppen enthaltenden Polyisocyanaten.**

(30) Priorität: **20.10.84 DE 3438527**

(43) Veröffentlichungstag der Anmeldung:
**30.04.86 Patentblatt 86/18**

(45) Bekanntmachung des Hinweises auf die
Patenterteilung:
**19.09.90 Patentblatt 90/38**

(84) Benannte Vertragsstaaten:
**BE DE FR GB IT NL**

(56) Entgegenhaltungen:
**EP-A-0 007 532**
**DE-A-1 814 972**
**US-A-3 906 019**

(73) Patentinhaber: **BAYER AG**
**D-5090 Leverkusen 1 Bayerwerk (DE)**

(72) Erfinder: **Kopp, Richard, Dr.**
**Bilharzstrasse 15**
**D-5000 Koeln 80 (DE)**
Erfinder: **Grögler, Gerhard, Dr.**
**von Diergardt Strasse 48**
**D-5090 Leverkusen (DE)**
Erfinder: **Hess, Heinrich, Dr.**
**Körnerstrasse 5**
**D-5090 Leverkusen 1 (DE)**
Erfinder: **König, Klaus, Dr.**
**Heymannstrasse 50**
**D-5090 Leverkusen (DE)**

## Beschreibung

Die Erfindung betrifft ein Verfahren zur Herstellung von feinteilig anfallenden, Harnstoffgruppen enthaltenden festen Polyisocyanaten durch Umsetzung von Harnstoffgruppenfreien organischen Polyisocyanaten mit Wasser, wobei das organische Polyisocyanat zunächst in einem großen Überschuß Wasser (bez. auf Polyisocyanatmenge) erfindungsgemäß in Gegenwart eines Schutzkolloides und gegebenenfalls eines Emulgators in eine stabile Emulsion bzw. Suspension überführt wird und die Umsetzung des Polyisocyanats mit dem Wasser, durch Zusatz von Katalysatoren und/oder gegebenenfalls Basen, zu den Harnstoffgruppen enthaltenden Polyisocyanaten durchgeführt wird.

Verfahren zur Herstellung von Harnstoffgruppen enthaltenden Polyisocyanaten sind an und für sich bekannt und Stand der Technik. In US 2 597 025 wird z.B. die Herstellung von reaktiven Harzmassen durch Umsetzung eines aromatischen Diisocyanats mit 0,1 bis 0,9 Mol Wasser pro Mol Diisocyanat in einem Lösungsmittel, welches sowohl das Wasser als auch das Diisocyanat zu lösen vermag und in Gegenwart von Wasser eine basische Reaktion zeigt, wie z.B. Pyridin, beschrieben. Die Harzmassen sind unter Feuchtigkeitsausschluß lagerstabil und härten in Gegenwart von Luftfeuchtigkeit innerhalb kürzester Zeit zu einem unschmelzbaren, unlöslichen Material, vermutlich einem Polyharnstoff. Die im Reaktionsmedium gelösten, Harnstoffgruppen enthaltenden Polyisocyanate werden nicht isoliert. Eine Herstellung und Isolierung von Harnstoffgruppen enthaltenden Polyisocyanaten definierter Zusammensetzung wird nicht angestrebt.

US 2 757 184 beschreibt die Herstellung vom substituierten Di(isocyanatophenyl)-harnstoffen durch Umsetzung von ca. 1 Mol Wasser mit 2 Mol Diisocyanat in inerten, Sauerstoffatome im Molekül enthaltenden Lösungsmitteln wie Ethern, Estern und Ketonen. In einigen Lösungsmitteln aus dieser Gruppe, wie z.B. Diethylether oder besser noch Di-i-propylether (s. DE—OS 3 419 429, Beispiel 4) werden Produkte erhalten, die in ihrem NCO-Gehalt dem theoretischen Wert praktisch entsprechen. Dieses Herstellungsverfahren weist aber den Nachteil auf, daß organische Lösungsmittel eingesetzt werden müssen, die wegen ihrer Flüchtigkeit und Oxidationsanfälligkeit (Ether) erhebliche Sicherheitsprobleme aufwerfen und deshalb hohe Verfahrenskosten verursachen. Ebenso neigen die in diesen Lösungsmitteln hergestellten Produkte, die in Form kleiner Kristallnadeln anfallen, bei der Aufarbeitung (Filtration) zum Agglomerieren, so daß anschließend ein teurer Mahlprozeß notwendig ist, wenn ein feinteiliges Produkt erhalten werden soll. Wird das Produkt z.B in einer Luftstrahlmühle gemahlen, so wird ein sehr feinteiliges Pulver erhalten, welches aber eine sehr geringe Schüttdichte (ca. 0.1—0,15 g/cm$^3$) aufweist und deshalb eines großes Lagervolumen beansprucht.

US 3 906 019 beschreibt die Herstellung von Di(isocyanatotolyl)harnstoffen, wobei einer der Reaktionspartner, Wasser oder Toluylendiisocyanat, im Überschuß eingesetzt wird und als Reaktionsmedium dient. Die entstehenden Di(isocyanatotolyl)harnstoffe sind in beiden Reaktionspartnern unlöslich und kristallisieren sofort nach ihrer Bildung aus. Dabei wird die Reaktion zwischen Wasser und Diisocyanat durch Zugabe von Verbindungen vom Lewis-Base- oder Lewis-Säure-Typ katalysiert. Bevorzugt wird Wasser im stöchiometrischen Überschuß eingesetzt und dient dann als Reaktionsmedium.

Vorzugsweise werden auf 20 bis 40 Teile Diisocyanat 5 bis 15 Teile Wasser eingesetzt. Die Produkte weisen einen NCO-Gehalt auf, der um ca. ≥10 rel.% gegenüber dem theoretischen NCO-Gehalt verringert ist. Als Ausbeuten werden bei den drei beschriebenen· Beispielen 60, 83 und 65 Gew.-% an Harnstoffdiisocyanat angegeben. Wenn man die Beispiel nachstellt, bei denen Wasser im Überschuß verwendet wird (Spalte 3, Zeile 1—16; Spalte 3, Zeile 45; Spalte 4, Zeile 7) so stellen sich gleich mehrere gewichtige Nachteil dieses Verfahrens heraus.

1. Die Umsetzung zwischen dem Diisocyanat und dem Wasser verläuft mit der angegebenen Wasser- und Katalysatormenge sehr exotherm; die Reaktionstemperatur kann bei dem beschriebenen kleinen Ansatz auf Seite 3, Zeile 1—15 nur durch Kühlen mit Eis-Kochsalzbad wie gefordert unter 40°C gehalten werden. Diese Tatsachse dürfte insbesondere bei größeren technischen Ansätzen zu gewaltigen Schwierigkeiten führen, da ja auch eine beträchtliche Menge an CO$_2$ innerhalb kurzer Zeit freigesetzt wird.

2. Das entstehende Harnstoffdiisocyanat backt sofort zusammen und scheidet sich an der Wand des Reaktionsgefäßes in Form einer dicken, harten Schicht ab. Diese kann nach der Umsetzung nur schwer aus dem Reaktionsgefäß entfernt werden, z.B. durch Kühlen des Reaktionsgefäßes einschließlich Produkt auf −60°C, wonach das spröde und brüchig gewordene Produkt dann in Form großer Brocken von der Gefäßwand entfernt werden kann. Nach dem Trocknen ist auf jeden Fall ein Mahlprozeß erforderlich.

3. Das bei der Umsetzung als Katalysator vorzugsweise eingesetzte Pyridin wird durch die im Patent beschriebene Aufarbeitung (Waschen mit Hexan oder Ethylacetat) nicht vollständig entfernt. Dadurch haftet den Produkten ein äußerst unangenehmer und lästiger Pyridingeruch an, der auch bei der weiteren Verwendung der Produkte durch Verdampfung und unkontrollierbare Katalyse für Probleme sorgan kann.

4. Die Ausbeuten liegen alle unter 85%, so daß ökologische Probleme bei der Beseitigung der abgetrennten flüssigen Phase auftreten können.

Der in US 3 906 019 beschriebene Herstellungsprozeß, der auf Toluylendiisocyanat beschränkt ist, bei dem zum erstenmal ein Wasserüberschuß als Reaktionsmedium eingesetzt wird, ist also in der offenbarten Form mit wesentlichen Nachteilen behaftet. Ziel der vorliegenden Erfindung wares, ein Verfahren zu Herstellung von festen Harnstoffgruppen enthaltenden Polyisocyanaten in Wasser als Reaktionsmedium zu entwickeln, bei dem diese Nachteile nicht mehr auftreten. Insbesondere wurde angestrebt, die Polyisocyanatteilchen in einer möglichst quantitativen Ausbeute und in einer solch feinteiligen Form herzustellen, daß die Produkte problemlos aufgearbeitet werden können und ein anschließender Mahlprozeß sich für die meisten Anwendungen erübrigt.

Aus EP—A1 7532 ist ein Verfahren zur Emulgierung organischer Polyisocyanate in Wasser in Gegenwart von Schutzkolloiden und Emulgatoren bekannt. Dieses Verfahren führt zu einer über mehrere Stunden stabilen Polyisocyanatemulsion, die zur Verleimung von Holz bzw. zur Herstellung von Holzwerkstoffen wie Spanplatten verwendet wird.

Gegenstand der Erfindung ist somit ein Verfahren zur Herstellung von Harnstoffgruppen enthaltenden, feinteilig anfallenden, festen Polyisocyanaten durch Umsetzung von Harnstoffgruppen-freien organischen Polyisocyanaten mit Wasser, dadurch gekennzeichnet, daß das organische Polyisocyanat in einem auf die Isocyanatmenge bezogenen großen Überschuß an Wasser in Gegenwart eines Schutzkolloids und gegebenenfalls eines Emulgators stabil emulgiert oder suspendiert wird und in dieser Emulsion oder Suspension die Reaktion des Polyisocyanats mit dem Wasser, unter Zusatz üblicher Katalysatoren, und gegebenenfalls unter Zusatz von Basen unter Aufrechterhaltung eines pH-Wertes ≥7 bis 10, zu den Harnstoffgruppen enthaltenden Polyisocyanaten ablaufen läßt.

Die anfängliche Dispergierung des Polyisocyanats in der wäßrigen Phase wird durch geeignete Emulgier- oder Dispergiermittel erleichtert.

Für die Durchführung des erfindungsgemäßen Verfahrens werden als Harnstoffgruppen-freie Ausgangspolyisocyanate aliphatische, cycloaliphatische, araliphatische, aromatische und heterocyclische Polyisocyanate eingesetzt, wie sie z.B. von W. Siefken in Justus Liebigs Annalen der Chemie, 562, Seiten 75 bis 136, beschrieben werden, beispielsweise solche der Formel

$$Q (NCO)_n$$

in der

n=2 bis 4, vorzugsweise 2, und

Q einen aliphatischen Kohlenwasserstoffrest mit 2 bis 18, vorzugsweise 6 bis 10 C-Atomen, einen cycloaliphatischen Kohlenwasserstoffrest mit 4 bis 15, vorzugsweise 5 bis 10 C-Atomen, einen aromatischen Kohlenwasserstoffrest mit 6 bis 15, vorzugsweise 6 bis 13 C-Atomen, oder einen araliphatischen Kohlenwasserstoffrest mit 8 bis 15, vorzugsweise 8 bis 13 C-Atomen,

bedeuten, z.B. Ethylen-diisocyanat, 1,4-Tetramethylendiisocyanat, 1,6-Hexamethylendiisocyanat, 1,12-Dodecandiisoyanat, Cyclobutan-1,3-diisocyanat, Cyclohexan-1,3- und -1,4-diisocyanat sowie beliebige Gemische ihrer Stereo-Isomeren, 1-Isocyanato-3,3,5-trimethyl-5-iso-cyanotomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder -1,4-phenylendiisocyanat, Perhydro-2,4'-und/oder -4,4'-diphenylmethandiisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-und/oder -2,2'-diisocyanat, Naphthylen-1,5-diisocyanat.

Ferner kommen beispielsweise erfindungsgemäß in Frage:

Triphenylmethan-4,4',4''-triisocyanat, Polyphenyl-polymethylen-polyisocyanate, wie sie durch Anilin-Formaldehyd-Kondensation und anschließende Phosgenierung erhalten und z.B. in den GB-Patentschriften 874 430 und 848 671 beschrieben werden, m- und p-Isocyanatophenylsulfonyl-isocyanate gemäß der US-Patentschrift 3 454 606, perchlorierte Arylpolyisocyanate, wie sie z.B. in der DE-Auslegeschrift 1 157 601 (US-Patentschrift 3 277 138) beschrieben werden, Carbodiimidgruppen aufweisende Polyisocyanate, wie sie in der DE-Patentschrift 1 092 007 (US-Patentschrift 3 152 162) sowie in den DE-Offenlegungsschriften 2 504 400, 2 537 685 und 2 552 350 beschrieben werden, Norbornan-Diisocyanate gemäß US-Patentschrift 3 492 330, Allophanatgruppen aufweisende Polyisocyanate, wie sie z.B. in der GB-Patentschrift 994 890, der BE-Patentschrift 761 626 und der NL-Patentanmeldung 7 102 524 beschrieben werden, Isocyanatgruppen aufweisende Polyisocyanate, wie sie z.B. in der US-Patentschrift 3 001 973 in den DE-Patentschriften 1 022 789, 1 222 067 und 1 027 394 sowie in den DE-Offenlegungsschriften 1 929 034 und 2 004 048 beschrieben werden, Urethangruppen aufweisende Polyisocyanate, wie sie z.B. in der BE-Patentschrift 752 261 oder in den US-Patentschriften 3 394 164 und 3 644 457 beschrieben werden, acylierte Harnstoffgruppen aufweisende Polyisocyanate gemäß der DE-Patentschrift 1 230 778, Biuretgruppen aufweisende Polyisocyanate, wie sie z.B. in den US-Patentschriften 3 124 605, 3 201 372 und 3 124 605 sowie in der GB-Patentschrift 889 050 beschrieben werden, durch Telomerisationsreaktionen hergestellte Polyisocyanate, wie sie z.B in der US-Patentschrift 3 654 106 beschrieben werden, Estergruppen aufweisende Polyisocyanate, wie sie z.B. in den GB-Patentschriften 965 474 und 1 072 956, in der US-Patentschrift 3 567 763 und in der DE-Patentschrift 1 231 688 genannt werden, Umsetzungsprodukte oder obengenannten Isocyanate mit Acetalen gemäß der DE-Patentschrift 1 072 385 und polymere Fettsäureester enthaltende Polyisocyanate gemäß der US-Patentschrift 3 455 883.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'- und/oder 2,2'-Diphenylmethandiisocyanat ableiten. Insbesondere bevorzugt ist das Tolylen-2,4-diisocyanat.

Das Polyisocyanat kann auch in Form einer (konzentrierten) Lösung in einem gegenüber Isocyanaten inerten, mit Wasser nicht mischbaren Lösungsmittel, vorzugsweise aliphatischen und aromatischen Kohlenwasserstoffen wie z.B. n-Hexan, Cyclohexan, Isooctan, Benzol, Toluol, Xylon oder ähnlichen, eingesetzt werden, doch ist dies nicht bevorzugt.

Es war für den Fachmann überraschend und nicht vorhersehbar, daß trotz einer chemischen Reaktion, bei der erhebliche Gasmengen entwickelt werden, die Herstellung von kleinen Teilchen mit annähernd kugelförmigem Aussehen möglich ist.

Bei der Durchführung der Erfindung wird in sämtlichen Fällen zunächst, beispielsweise durch einfaches Rühren, eine Lösung aus Wasser, einem geeigneten Schutzkolloid und gegebenenfalls einem Emulgator hergestellt. Diese zwei bzw. drei Komponenten bilden die wäßrige Phase des Verfahrens.

Als Emulgatoren können in der wäßrigen oder kontinuierlichen Phase nicht-ionische, anionische oder kationische oberflächenaktive Mittel vom HLB (Hydrophilie-Lipophobie-Balance)-Bereich 10 bis 18, vorzugsweise 13 bis 16, verwendet werden. Der HLB-Wert wird in einer Firmenschrift der Atlas-Chemie, 4300 Essen, aus dem Jahre 1968, beschrieben. Es gibt zahlreiche oberflächenaktive Mittel, die in diesen Bereich fallen, beispielsweise Verbindungen wie Natriumpropyl-naphthalinsulfonat, Polyoxyethylensorbitoleat-laurat, ethoxylierte Nonylphenole, Polyethylenglykolether linearer Alkohole oder Polyethylenglykolester linearer Carbonsäuren. Das oberflächenaktive Mittel kann nicht nur wie beschrieben der wäßrigen Phase (bevorzugt), sondern auch der organischen Phase beigegeben werden. Ohne besondere Angaben hinsichtlich der Phase, in welche das oberflächenaktive Mittel eingegebenen wird, liegt eine Verteilung dieses Mittels zwischen den Phasen nach Phasenvermischung vor, je nach den relativen Löslichkeiten. Die Verwendung eines oberflächenaktiven Mittels kann auch entfallen, wenn bei der Bildung der Dispersion ausreichend hohe. Schergeschwindigkeiten angewandt werden oder das Schutzkolloid eine ausreichende Wirkung auch als Emulgator aufweist. Gemäß der bevorzugten Ausführungsform der Erfindung arbeitet man mit oberflächenaktiven Emulgatoren. Die Konzentration dieses Mittels liegt in dem System am günstigsten zwischen 0,001 und 3,0 Gew.-%, bezogen auf die wäßrige Phase. Höhere Konzentrationen können angewandt werden, ohne die Dispergierbarkeit zu erhöhen.

Wesentlicher, erfindungsgemäß einzusetzender Bestandteil der wäßrigen oder kontinuierlichen Phase ist ein Schutzkolloid, welches aus einer Vielzahl derartiger Materialien ausgewählt sein kann. Als Beispiel für verwendbare Schutzkolloide seien genannte; Polyacrylate, Methylcellulose, Polyvinylalkohol, Polyacrylamid und Poly(methylvinylether/Maleinsäureanhydrid). Bevorzugt sind Schutzkolloide auf Basis von Polyvinylalkohol und Methylcellulose. Die Menge an Schutzkolloid hängt von verschiedenen Faktoren, wie dem Molekulargewicht, der Art und Wirksamkeit innerhalb der Medien, der Verträglichkeit und dergleichen ab. Das Schutzkolloid sollte der wäßrigen Phase vor Zusatz des organischen Polyisocyanats zugegeben werden. Weniger bevorzugt ist es, das Schutzkolloid dem System im Anschluß an den Zusatz des organischen Polyisocyanats oder nach der Dispergierung zusetzen. Außerdem kann man einen Teil des Schutzkolloids vor der Zugabe des organischen Polyisocyanats und einem weiteren Teil nach der Dispergierung zusetzen. Man verwendet 0,01 bis 5,0 Gew.-%, bevorzugt 0,01 bis 0,5 Gew.-% bezogen auf die wäßrige Phase.

Die Menge an organischem Polyisocyanat, bezogen auf das gesamte Reaktionsgemisch, kann zwischen 1 und 65 Gew.-% betragen. Die bevorzugte Menge an organischem Polyisocyanat beträgt 10 bis 30 Gew.-%, bezonders bevorrzugt 15 bis 25 Gew.-%.

Zur Ausbildung der Dispersion wird das organische Polyisocyanat der wäßrigen Phase, gegebenenfalls unter Rühren, zugesetzt. Hierbei wendet man geeignete Dispergiermittel zum Dispergieren einer Flüssigkeit in einer anderen an. Es können beispielsweise Ultraschall-Zerkleinerungsgeräte oder Geräte verwendet werden, an denen die Stoffströme durch Düsen mit hoher Geschwindigkeit gegeneinander oder gegen Prallvorrichtungen geschleudert werden. Insbesondere sind jedoch solche Vorrichtungen geeignet, bei denen die zu zerkleinernde Dispersion unter hoher Beschleunigung und Scherwirkung durch Gitter oder Schlitze geschleudert wird. Dies wird etwa dadurch erreicht, daß das Material mit nach dem Rotor-Stator-Prinzip arbeitenden Maschinen durch z.B. aufeinanderliegende gegenläufige Lochplatten, durch rotierende geschlitzte Zylinder, zwischen denen nur eine enger Spalt liegt,. oder durch gegenläufige rotierende Zahnkränze gedrückt wird. Solche Geräte sind marktüblich und werden z.B. als Mischsirene bezeichnet. Geräte, die nach dem Rotor-Stator-Prinzip arbeiten, werden bevorzugt eingesetzt.

Gerät dieser Art werden z.B. unter der Bezeichnung Supraton®, Condix® Mischer, oder Ultraturrax® handelsüblich angeboten. Besonders gut geeignete, derartige Mischaggregate gestatten die Durchmischung von flüssigen Materialien unter einer Leistung im Mischkopfbereich von 15 bis 250 Watt/cm³.

Sobald die gewünschte Tröpfchengröße erreicht ist, wird die intensive mechanische Dispergierung (vorzugsweise bei +10 bis +40°C, insbesondere bei Raumtemperatur) abgebrochen. Diese liegt bei 0,5 bis 200 µm, insbesondere 0,5 bis 20 µm, und bestimmt die Teilchengröße der gewünschten, festen Harnstoffgruppen enthaltenden Polyisocyanate. Während der zweiten Phasen des Verfahrens muß nur noch mäßig bewegt werden.

Nach dem Herstellen der feinteiligen Dispersion läuft die Umsetzung zwischen organischem Polyisocyanat und Wasser bei 10 bis 80°C, vorzugsweise 20 bis 40°C, vorzugsweise unter Normaldruck, ab. Jedoch kann auch unter Zuhilfenahme von geeigneten Vorrichtungen unter einem erhöhten Druck, der sich auch durch das freiwerdende $CO_2$ selbst aufbauen kann, oder unter einem vermindertem Druck gearbeitet werden.

Die entstehenden, Harnstoffgruppen enthaltenden Polyisocyanate sind in Wasser schwer löslich und entziehen sich so im allgemeinen einer Weiterreaktion der noch vorhandenden NCO-Gruppen mit dem Wasser, so daß im allgemeinen von den eingesetzten organischen Polyisocyanaten nur eine NCO-Gruppe pro Molekül an der Harnstoffgruppenbildung teilnimmt. Die NCO-Gehalte der nach dem erfindungsgemäßen Verfahren erhaltenen Produkte liegen in den meisten Fällen nur geringfügig unterhalb des bereichneten NCO-Gehalts. Jedoch sind auch Produkte mit einem gegenüber der Theorie wesentlichen verringertem NCO-Gehalt, bei denen also eine Oligoharnstoffbildung eingetreten ist, für gewisse Anwendungen noch interessant.

Bei höheren Reaktionstemperaturen steigt die Löslichkeit des Harnstoffgruppen enthaltenden Polyisocyanats an, so daß eine Weiterreaktion mit Wasser unter Bildung von polymeren oder oligomeren Harnstoffen erfolgen kann. Im allgemeinen sollte deshalb die Reaktionstemperatur zwischen 20 und 40°C gehalten werden. Auch muß die Menge an Schutzkolloiden bzw. Dispergierhilfsmitteln innerhalb der angegebenen Mengengrenzen gehalten werden, um nicht eine zu starke Hydrophilierung und somit eine Weiterreaktion der NCO-Gruppen im gebildeten Harnstoffdiisocyanat auszulösen.

Zur Beschleunigung der Reaktion zwischen Wasser und organischem Polyisocyanat werden die in der Polyurethanchemie üblicherweise eingesetzten Katalysatoren, wie z.B. tertiäre Amine (N,N-Dimethylbenzylamin, Triethylamin, Pyridin, Bis(dimethylaminoethyl)ether) oder Metallorgan. Verbindungen (Tributylzinnacetat, Di-n-butyl-zinn-diacetat, Sn(II)dioctoat, Dibutylzinn-dilaurat) in katalytische wirksamen Mengen eingesetzt werden. Der Katalysatorzusatz erfolgt vorzugsweise nach der Emulsionsbildung. Die Katalysatormenge wird vorzugsweise so gewählt, daß die Umsetzung nach ca. 2 bis 8 Stunden beendet ist, und daß die entwickelte Reaktionswärme das Gemisch nicht über 35°C erwärmt, so daß eine externe Kühlung entfallen kann. Die Menge des Katalysators liegt im allgemeinen bei 0,01—10, vorzugsweise 0,1—4 Gew.-% bezogen auf Polyisocyanat.

Die Reaktion zwischen organischem Polyisocyanat und Wasser ist an der $CO_2$-Entwicklung zu beobachten. Diese kann, insbesondere bei höheren Emulgator- und Schutzkolloid-Konzentrationen, zu beträchtlicher Schaumbildung und/oder Volumenausdehnung führen, welche die Verarbeitung des Reaktionsgemisches stören. Eine Möglichkeit bietet die chemische Bindung von $CO_2$ durch Durchführung der Reaktion bei einem pH $\geq$7 bis 10 (bevorzugt 8,5 bis 9,5), wobei der pH-Wert durch Basenzugabe (z.B. wäßrige Natronlauge) in dem genannten Bereich gehalten wird. Zusätzlich oder alternativ kann ein handelsübliches Antischaummittel wie z.B. Tributylphosphat zugegeben werden.

Nach dem Ende der Reaktion, welches am Aufhören der $CO_2$-Entwicklung zu beobachten ist, wird die Feststoffsuspension über ein geeignetes Filter abgesaugt, mit Wasser gewaschen, und dann gegebenenfalls mit einem gegenüber Isocyanaten inerten, gegebenenfalls eine gewisse Wasserlöslichkeit besitzenden Lösungsmittel wie z.B. Ethylacetat oder Aceton, nochmals gewaschen und in einem Trockenschrank, vorzugsweise im Vakuum, bei niedrigen Temperaturen (50°C) getrocknet.

Wenn tertiäre Amine als Katalysatoren mitverwendet werden, können diese durch Zugabe einer äquivalenten Säuremenge in Form ihrer Salze vollständig in die wäßrige Phase überführt werden, so daß sie im festen Polyisocyanat nach dessen Aufarbeitung nicht mehr vorhanden sind. Auch andere an sich bekannte Verfahren zur Entfernung von mitverwendeten Katalysatoren sind möglich. Gegebenenfalls kann die Umsetzungsreaktion auch durch Inaktivieren des Katalysators zu einem Zeitpunkt beendet werden, der vor dem eigentlichen Reaktionsende liegt.

Bei der Anwendung des erfindungsgemäßen Verfahrens werden Produkte mit nahezu idealer Kugelform erhalten. Die Teilchendurchmesser liegen im allgemeinen bei 1 bis 20 µm.

Beispiele
Eingesetzte Ausgangsverbindungen
Schutzkolloid 1:     Polyvinylalkohol
                     Mowiol® 26/88 der Fa. Hoechst,
                     Frankfurt/Main

Schutzkolloid 2:     Methylcellulose
                     (Cellulosemethylether)
                     Tylose® MH 50 der Fa. Hoechst,
                     Frankfurt/Main

| Emulgator 1: | Atlox® 4851 B der Fa. Atlas Chemie, Essen Mischung von anionaktiven und nichtionogenen Tensiden, HLB-Wert 13.2 |
|---|---|
| Emulgator 2: | handelsübliches Addukt aus 10 Mol Ethylenoxid und 1 Mol Nonylphenol (HLB 13,9). |

Allgemeine Beschreibung der Versuchsdurchführung zur Herstellung der Harnstoffdiisocyanate

Die angegebene Wassermenge (vorzugsweise demineralisiertes Wasser) wird mit der angegebenen Schutzkolloidmenge (vorzugsweise in Form einer ca. 10 %igen wäßrigen Lösung) und der angegebenen Emulgatormenge (vorzugsweise in Form einer 1 %igen Lösung) und eventuellen weiteren Zusätzen bei Raumtemperatur in einem 2 l Becherglas homogen vermischt.

Dann wird die abgewogene Menge des Ausgangspolyisocyanats zugesetzt und die zweiphasige Mischung anschließend mit einem Ultraturrax-Rührer (Typ T 45/N der Fa. IKA-Werk, Staufen i. Breisgau) intensiv vermischt (Geschwindigkeitsregler auf ca. 1/3 bis 1/2 der vollen Leistung aufgedreht) bis eine stabile Emulsion entstanden ist (im allgemeinen 3 Minuten). Diese wird in einen 2 l-Sulfierbecher mit Planschliffdeckel überführt und mit einem üblichen Blattrührer weitergerührt.

Nach Zugabe des angegebenen Katalysators wird die bei der Reaktion eintretende $CO_2$-Entwicklung mit einer Gasuhr verfolgt. Nach Beendigung der $CO_2$-Entwicklung wird mit der angegebenen Menge an 1 N HCl neutralisiert, die Suspension nach ca. 10 Minuten Rühren abgesaugt, der Filterrückstand mehrmals mit Wasser gewaschen und bei 50°C im Vakuumtrockenschrankt getrocknet.

Beispiel 1
(s. allgemeine Beschreibung der Versuchsdurchführung)

| wäßrige Phase: | 500 g Wasser 0,05 g Emulgator 1 0,25 g Schutzkolloid 1 |
|---|---|
| Polyisocyanat: | 150 g 2,4-Toluylendiisocyanat |
| Katalysator: | 1 g Pyridin |
| Neutralisation: | 13 ml 1 N HCl |

Es wurden 134,3 g (96,8%) eines feinpulvrigen Produktes mit einem NCO-Gehalt von 23,02 Gew.-% und einer Teilchengröße ≤20 µm erhalten.

Bestimmung des NCO-Gehaltes in den Harnstoffdiisocyanaten

Eine abgewogene Menge von ca. 1 bis 2 g des zu titrierenden Polyisocyanates wird zu 20 ml 1 N-Dibutylamin-Lösung in Chlorbenzol gegeben und für ca. 2 Minuten unter gelegentlichen Umschwenken auf ca. 80°C erhitzt. Hiernach werden 30 ml Dimethylformamid zugegeben und der Überschuß an Dibutylamin mit 1 N HCl zurücktitriert.

Beispiel 2

| wäßrige Phase: | 500 g Wasser 0,5 g Schutzkolloid 1 |
|---|---|
| Polyisocyanat: | 150 g 2,4-Toluylendiisocyanat |
| Katalyse: | 2,0 g Pyridin |
| Neutralisation: | 26,0 ml 1 N HCl |

Es wurden 132,6 g (95,5%) eines pulverförmigen Produktes mit einem NCO-Gehalt von 22,43 Gew.-% erhalten, welches zum allergrößten Teil aus kugelförmigen Teilchen mit einem Durchmesser von 3 bis 7 µm bestand.

Beispiel 3
(Vergleichsbeispiel—ohne Schutzkolloid)

| | |
|---|---|
| wäßrige Phase: | 500 g Wasser |
| | 0,1 g Emulgator 2 |
| Polyisocyanat: | 150 g 2,4-Toluylendiisocyanat |
| Katalysator: | 2,0 g Pyridin |
| Neutralisation: | 26,0 ml 1 N HCl |

Im Verlaufe der Reaktion lagerte sich das entstehende Harnstoffgruppen-haltige Polyisocyanat in Form eines sehr festen Kuchens an der Innenwand des Reaktionsgefäßes ab. Dieser konnte z.B. nach Abgießen der wäßrigen Phase durch Kühlen des Kolbens samt Inhalt auf −60°C entfernt werden und lieferte nach dem Trocknen und Zerkleinern im Mörser 120,1 g (86,5%) eines Produktes mit einem NCO-Gehalt von 22,80 Gew.-%. Die zerkleinerten Partikel besaßen kein kugelähnliches Aussehen.

Beispiel 4
(Vergleichsbeispiel gemäß Beispiel aus US 3 906 019 in Spalte 3, Zeile 1—16)

300 g 2,4-Toluylendiisocyanat,
100 g Wasser und
7 g Pyridin wurden in einem 2 l-Planschliff-Reaktionsgefäß mit Planschliffdeckel intensiv miteinander verrührt. Nur unter intensiver Eis-Kochsalz-Kühlung gelang es, die Reaktionstemperatur auf nicht mehr als 42°C steigen zu lassen. Die Umsetzung war nach ca. 1 Stunde beendet. Nach Reaktionsende wurde des überstehende Wasser abgegossen und das fest an der Innenwand des Reaktionsgefäßes hängende Produkt mittels eines Spatels entfernt, abgesaugt und 2×in Hexan aufgeschlämmt, abgesaugt und getrocknet.

Ausbeute: 238,3 g=85,8% in Form sehr grober, bis zu 2 cm dicker Brocken
NCO-Gehalt: 21,66 Gew.-%

Beispiel 5
(Vergleichsbeispiel gemäß Beispiel 1 aus US 3 906 019)

325 g 2,4-Toluylendiisocyanat,
50 g Wasser und
1,75 g Pyridin

wurden in einem offenen Becherglas intensive vermischt. Die Temperatur des Reaktionsgemisches wurde durch Zugabe von ca. 50 g Eis auf 30 bis 40°C gehalten. Die Reaktion war nach ca. 30 Minuten beendet.

Das Produkt wurde abgesaugt, 2×mit Hexan gewaschen und bei 50°C im Vakuumtrockenschrank getrocknet.

Ausbeute: 210,5 g=70% in Form sehr grober, bis zu 1 cm dicker Brocken
NCO-Gehalt: 24,5 Gew.-%
Das Produkt wies einen intensiven Geruch nach Pyridin auf.

Beispiel 6

| | |
|---|---|
| wäßrige Phase: | 500 g Wasser |
| | 0,75 g Schutzkolloid 1 |
| | 0,15 g Emulgator 2 |
| Polyisocyanat: | 150 g 2,4-Toluylendiisocyanat |
| Katalysator: | 2,0 g Pyridin |
| Neutralisation: | 26,0 ml 1 N HCl |

Es wurden 111,68 g (80,5%) (Verluste bei Filtration durch Anteil an extrem feinteiligen Feststoffen, die vom Filter nicht zurückgehalten wurden) eines sehr feinteiligen, agglomeratfreien Pulvers mit einem NCO-Gehalt von 19,40 Gew.-% erhalten, welches aus ideal runden Kugeln mit einem Durchmesser ≤15 μm bestand.

Beispiel 7

| | | |
|---|---|---|
| wäßrige Phase: | 500 g Wasser | |
| | 0,25 g Schutzkolloid 2 | |
| | 0,05 g Emulgator 1 | |
| Polyisocyanat: | 150 g 2,4-Toluylendiisocyanat | |
| Katalysator: | 0,5 g Pyridin | |
| Neutralisation: | 6,5 ml 1 N HCl | |

Es wurden 134,3 g (96,8%) eines feinteiligen Produktes mit einem NCO-Gehält von 22,90 Gew.-% und einer mittleren Teilchengröße von ca. 10 µm erhalten.

Beispiel 8

| | | |
|---|---|---|
| wäßrige Phase: | 500 g Wasser | |
| | 0,05 g Emulgator 1 | |
| | 0,25 g Schutzkolloid 1 | |
| Polyisocyanat: | 150 g 2,4-Tolylendiisocyanat | |
| Katalysator: | jeweils 2 g der untenstehenden Verbindungen | |
| Neutralisation: | jeweils mit der angegebenen Menge 1 N HCl | |

Die Produkte fielen als feine Pulver mit einer Teilchengröße von ca. 15 µm an.

| Vers. | Katalysator | ml 1 N HCl | Ausbeute g | % | % NCO des Produktes |
|---|---|---|---|---|---|
| A | Triethylamin | 20,69 | 133,0 | 95,8 | 21,81 |
| B | Triethylendiamin | 22,89 | 133,4 | 96,1 | 22,30 |
| C | Dimethylbenzylamin | 15,02 | 132,3 | 95,2 | 22,55 |
| D | Pyridin | 26,00 | 129,02 | 93,0 | 22,60 |
| E | N-Methylmorpholin | 20,58 | 132,5 | 95,5 | 24,0 |
| F | Bis(dimethylamino-ethylether) | 17,43 | 135,4 | 97,6 | 21,95 |
| G | Tetramethylharnstoff | 0,10 | 134,7 | 97,1 | 24,30 |
| H | UL 1 der Fa.Witco Dibutyl-Sn-dimercaptid | 0,20 | 134,6 | 97,0 | 23,93 |
| I | Sn(II)octoat | 0,00 | 132,2 | 95,2 | 23,90 |

| | Beispiel 9 | | | |
| --- | --- | --- | --- | --- |
| | A | B | C | |
| wäßrige Phase: | 500 g | 700 g | 1000 g | Wasser |
| | 0,25 g | 0,25 g | 0,25 g | Schutzkolloid 1 |
| | 0,05 g | 0,05 g | 0,05 g | Emulgator 1 |
| Polyisocyanat | 150 g | 150 g | 150 g | 2,4-Toluylen-diisocyanat |
| Katalysator | 1,0 g | 1,0 g | 1,0 g | Pyridin |
| Neutralisation | 13,0 ml | 13,0 ml | 13,0 ml | 1 N HCl |
| Ausbeute | 134,3 g (96,8 %) | 134,9 g (97,2 %) | 133,6 g (96,3 %) | |
| NCO-Gehalt | 23,02 | 24,0 | 23,19 | Gew.-% |
| Teilchengröße | ≤20 μm | ≤10 μm | ≤10 μm | |

**Beispiel 10**

wäßrige Phase: 500 g Wasser
0,25 g Schutzkolloid 1
0,05 g Emulgator 1

Polyisocyanat: 150 g eines bei der Phosgenierung von Anilin-Formaldehyd-Kondensationsprodukten anfallenden Roh-Polyisocyanates mit einem NCO-Gehalt von 31 Gew.-%, Viskosität ≤100 mPas bei 25°C

Katalysator: 2,0 g Pyridin

Neutralisation: 26,0 ml 1 N HCl

Es wurden 130,6 g (ca. 91,8%) eines sehr feinteiligen, agglomeratfreien Pulvers mit einem NCO-Gehalt von 8,48 Gew.-% und einer Teilchengröße von 6 bis 26 μm erhalten, welches vollständig in Form ideal runder Kugeln vorlag.

**Beispiel 11**

wäßrige Phase: 500 g Wasser
0,25 g Schutzkolloid 1
0,05 g Emulgator 1

Polyisocyanat: 120 g eines Gemisches aus ca.
35 Gew.-% 4,4'-Diisocyanatodiphenylmethan und ca.
65 Gew.-% 2,4'-Diisocyanatodiphenylmethan

Katalysator: 1,0 g Pyridin

Neutralisation: 26,0 ml 1 N HCl

Es wurden 131,6 g (ca. 92,5%) eines sehr feinteiligen, agglomeratarmen Pulvers mit einem NCO-Gehalt von 5,80 Gew.-% und einer Teilchengröße von 5 bis 20 μm erhalten, welches vollständig in Form ideal runder Kugeln vorlag.

**Beispiel 12**

wäßrige Phase: 500 g Wasser
0,75 g Schutzkolloid 1
0,1 g Emulgator 1

Polyisocyanat: 150 g Isophorondiisocyanat

Katalysator: 1,0 g UL 1 (s. Beispiel 8)

Neutralisation: 0,2 ml 1 N HCl

9

Es wurden 72,4 g (51,3%) (Verluste bei Aufarbeitung) eines Produktes mit einem NCO-Gehalt von 10,10 Gew.-% und einer Teilchengröße bis ca. 200 µm erhalten, wobei der größte Teil in Form von Kugeln mit einem Durchmesser ≤80 µm vorlag.

Beispiel 13
Mitverwendung eines Entschäumers

| wäßrige Phase: | 500 g Wasser |
| | 0,05 g Emulgator 1 |
| | 0,25 g Schutzkolloid 1 |
| | 0,09 g Tributylphosphat als Entschäumer |
| Polyisocyanat: | 150 g 2,4-Toluylendiisocyant |
| Katalysator: | 1,0 g Pyridin |
| Neutralisation: | 13 ml 1 N HCl |

Es wurden 131,3 g (94,60%) eines Produktes mit einem NCO-Gehalt von 24,0 Gew.-% und einer Teilchengröße ≤13 µm erhalten, welches nur sehr wenige agglomerierte Teilchen aufwies. Es wurde ein gegenüber Beispiel 1 deutlich vermindertes Aufsteigen des durch die $CO_2$-Entwicklung entstehenden Schaumes beobachtet.

Beispiel 14
Herstellung eines Elastomeren aus einem höher molekularen Polyetherdiamin mit aromatischen Aminogruppen und dem Harnstoffgruppen-haltigen Polyisocyanat gemäß Beispiel 1.

Herstellung des höhermolekularen, aromatischen Polyamins (Ausgangsmaterial)
1 Mol eines linearen Polypropylenetherglykols der OH-Zahl 56 und 2 Mol Toluylen-2,4-diisocyanat wurden durch 4-stündiges Erhitzen auf 80°C in ein NCO-Prepolymer (3,58% NCO) überführt. 810 g des 45°C warmen NCO-Prepolymers wurden unter intensivem Rühren so zu einer gekühlten Lösung von 52,2 g Kaliumhydroxid und 500 ml Wasser und 300 ml Aceton gegeben (NCO:OH$^{\ominus}$-Verhältnis=1:1,35), daß eine Innentemperatur von 25°C nicht überschritten wurde. 30 Minuten wurde bei dieser Temperatur weitergerührt und dann 2 Stunden zum Rückfluß erhitzt. Nach 30-minütigem Stehen wurde die untere, wäßrige Salzlösung aus dem zweiphasigen Reaktionsgemisch abgetrennt und verworfen. Die obere Phase wurde bei 20 mbar/80°C und dann bei 1 mbar/100°C von Wasser- und Acetonresten befreit. Durch Absaugen des 60°C warmen Produkts über eine Drucknutsche (3 bar Überdruck) wurden geringe Reste Salz abgetrennt und das Polyetheramin mit einer NH-Zahl von 48,4 isoliert.

Herstellung des Elastomeren
100 Teile des höhermolekularen aromatischen Polyamins wurden mit 20 Teilen des feinteiligen Polyisocyanates aus Beispiel 1 intensiv vermischt, sofort im Wasserstrahlvakuum entgast, in eine cas. 20 cm×20 cm×0,5 cm große, offene, kalte Form gegossen und in einem Heizschrank bei 120°C 4 Stunden ausgeheizt. Tabelle 1 zeigt die mechanischen Werte der erhaltenen Elastomeren. Die entgaste Mischung aus aromatischem Aminopolyether und Polyisocyanat zeigte beim langsamen Aufheizen (ca. 10 K/min) bei ca. 88°C einen plötzlichen, starken Viskositätsanstieg unter Bildung einer plastischen bis elastischen Phase.

TABELLE 1

| | | |
|---|---|---|
| Zugfestigkeit | DIN 53 504 | 11,3 MPa |
| Reißdehnung | DIN 53 504 | 200% |
| Weiterreißfestigkeit | DIN 53 515 | 35,7 kN/m |
| Shore Härte | DIN 53 505 | 92 A 42 D |
| Elastizität | DIN 53 512 | 51% |

**Patentansprüche**

1. Verfahren zur Herstellung von Harnstoffgruppen enthaltenden, feinteilig anfallenden, festen Polyisocyanaten durch Umsetzung von Harnstoffgruppen-freien organischen Polyisocyanaten mit Wasser, dadurch gekennzeichnet, daß man

das organische Polyisocyanat in einem auf die Isocyanatmenge bezogenen großen Überschuß an Wasser,

in Gegenwart von 0,01 bis 5 Gew.-%, bezogen auf die wäßrige Phase, eines Schutzkolloids und gegebenenfalls 0,001 bis 3 Gew.-%, bezogen auf die wäßrige Phase, Emulgatoren stabil emulgiert oder suspendiert und in dieser Emulsion oder Suspension die Reaktion des Polyisocyanats mit dem Wasser, unter Zusatz üblicher Katalysatoren und gegebenenfalls unter Zusatz von Basen unter Aufrechterhaltung eines pH-Wertes ≥7 bis 10, zu den Harnstoffgruppen enthaltenden Polyisocyanaten ablaufen läßt, anschließend den Feststoff abtrennt und trocknet.

2. Verfahren nach Anspruch 1, dadurch gekennzeichnet, daß man als Schutzkolloid Polyvinylalkohol, Methylcellulose, Polyacrylate, Polyacrylamide und/oder Polymethylvinylether/Maleinsäreanhydrid-Copolymere verwendet.

3. Verfahren nach Ansprüchen 1 und 2, dadurch gekennzeichnet, daß man als Schutzkolloide 0,01 bis 0,5 Gew.-% Polyvinylalkohol und/oder Methylcellulose verwendet.

4. Verfahren nach Ansprüchen 1 bis 3, dadurch gekennzeichnet, daß man als Emulgatoren nichtionische, anionische und/oder kationische oberflächenaktive Mittel mit einem HLB-Wert (Hydrophile-Lipophobie-Balance-Wert) von 10 bis 18 verwendet.

5. Verfahren nach Ansprüchen 1 bis 4, dadurch gekennzeichnet, daß man 1—65 Gew.-%, vorzugsweise 10—30 Gew.-%, an organischem Polyisocyanat, bezogen auf das gesamte Reaktionsgemisch, verwendet.

6. Verfahren nach Ansprüchen 1 bis 5, dadurch gekennzeichnet, daß man als Polyisocyanat das 2,4-Toluylendiisocyanat verwendet.

7. Verfahren nach Ansprüchen 1 bis 6, dadurch gekennzeichnet, daß man das Polyisocyanat zusammen mit dem Schutzkolloid und gegebenenfalls Emulgator intensiv mechanisch bis zur Tröpfchenbildung mit einer Teilchengröße von 0,5 bis 20 µm dispergiert und die wäßrige Dispersion bei Temperaturen von vorzugsweise 20 bis 40°C zur Bildung des Harnstoffdiisocyanats abreagieren läßt.

8. Verfahren nach Ansprüchen 1 bis 7, dadurch gekennzeichnet, daß man den Katalysator vor der Aufarbeitung des festen Harnstoffdiisocyanats desaktiviert oder entfernt.

## Revendications

1. Procédé de production de polyisocyanates solides ein fines particules, contenant des groupes urée, par réaction avec l'eau de polyisocyanates organiques dépourvus de groupes urée, caractérisé en ce qu'on forme une émulsion ou une suspension stable du polyisocyanate organique dans de l'eau en grand excès par rapport à la quantité d'isocyanate,

en présence de 0,01 à 5% en poids, par rapport à la phase aqueuse, d'un colloïde protecteur et le cas échéant, de 0,001 à 3% en poids, par rapport à la phase aqueuse, d'émulsionnants et on laisse se dérouler dans cette émulsion ou suspension la réaction du polyisocyanate avec l'eau, avec addition de catalyseurs classiques et, le cas échéant, avec addition de bases en entretenant une valeur de pH égale ou supérieure à 7—10 pour former les polyisocyanates contenant des groupes urée, après quoi on sépare la matière solide et on la séche.

2. Procédé suivant la revendication 1, caractérisé en ce qu'on utilise comme colloïde protecteur un polymère d'alcool vinylique, la méthylcellulose, des polyacrylates, des polyacrylamides et/ou des copolymères d'éther de méthyle et vinyle et d'anhydride d'acide maléique.

3. Procédé suivant les revendications 1 et 2, caractérisé en ce qu'on utilise comme colloïdes protecteurs 0,01 à 0,5% en poids de polymère d'alcool vinylique et/ou de méthylcellulose.

4. Procédé suivant les revendications 1 à 3, caractérisé en ce qu'on utilise comme émulsionnants des agents tensio-actifs non ioniques, anioniques et/ou cationiques ayant une valeur EHL (valeur de l'Equilibre Hydrophile-Lipophobe) de 10 à 18.

5. Procédé suivant les revendications 1 à 4, caractérisé en ce qu'on utilise 1 à 65% en poids, de préférence 10 à 30% en poids de polyisocyanate organique, par rapport au mélange réactionnel total.

6. Procédé suivant les revendications 1 à 5, caractérisé en ce qu'on utilise comme polyisocyante le 2,4-diisocyanatotoluène.

7. Procédé suivant les revendications 1 à 6, caractérisé en ce qu'on disperse énergiquement par des moyens mécaniques le polyisocyanate conjointement avec le colloïde protecteur et, le cas échéant, l'émulsionnant jusqu'à formation de gouttelettes ayant un diamètre de 0,5 à 20 µm et on fait réagir la dispersion aqueuse à des températures allant de préférence de 20 à 40°C pour former l'urée-diisocyanate.

8. Procédé suivant les revendications 1 à 7, caractérisé en ce qu'on désactive ou élimine le catalyseur avant le traitement final de l'urée-diisocyanate solide.

## Claims

1. A process for the production of finely divided, solid polyisocyanates containing urea groups by reaction of organic polyisocyanates free from urea groups with water, characterized in that the organic polyisocyanate is stably emulsified or suspended in a large excess of water, based on the quantity of isocyanate,

in the presence of 0.01 to 5% by weight, based on the aqueous phase, of a protective colloid and optionally 0.001 to 3% by weight, based on the aqueous phase, of emulsifiers

and the reaction of the polyisocyanate with the water to form the polyisocyanates containing urea groups is carried out in the resulting emulsion or suspension with addition of typical catalysts and optionally with addition of bases at a pH value kept in the range from ≥7 to 10, after which the solid is separated off and dried.

2. A process as claimed in Claim 1, characterized in that polyvinyl alcohol, methyl cellulose, polyacrylates, polyacrylamides and/or polymethyl vinyl ether/maleic anhydride copolymers is/are used as the protective colloid.

3. A process as claimed in Claims 1 and 2, characterized in that 0.01 to 0.5% by weight polyvinyl alcohol and/or methyl cellulose is used as the protective colloid.

4. A process as claimed in Claims 1 to 3, characterized in that nonionic, anionic and/or cationic surfactants having an HLB (hydrophilic-lipophobic balance value) of 10 to 18 are used as the emulsifiers.

5. A process as claimed in Claims 1 to 4, characterized in that 1 to 65% by weight and preferably 10 to 30% by weight organic polyisocyanate, based on the reaction mixture as a whole, is used.

6. A process as claimed in Claims 1 to 5, characterized in that 2,4-tolylene diisocyanate is used as the polyisocyanate.

7. A process as claimed in Claims 1 to 6, characterized in that the polyisocyanate is intensively mechanically dispersed together with the protective colloid and, optionally, emulsifier to form droplets having a particle size of 0.5 to 20 µm and the aqueous dispersion is left to react off at temperatures of preferably 20 to 40°C to form the urea diisocyanate.

8. A process as claimed in Claims 1 to 7, characterized in that the catalyst is deactivated or removed before the solid urea diisocyanate is worked up.